Europäisches Patentamt

European Patent Office

Office européen des brevets

(19)

(11) Numéro de publication: **0 085 589**
**B1**

(12) **FASCICULE DE BREVET EUROPEEN**

(45) Date de publication du fascicule du brevet:
**11.03.87**

(21) Numéro de dépôt: **83400078.8**

(22) Date de dépôt: **13.01.83**

(51) Int. Cl.⁴: **C 12 N 5/02,** C 12 N 5/00,
A 61 K 35/78

(54) **Procédé de préparation de suspensions de cellules fraîches stabilisées destinées à l'usage pharmaceutique, cosmétique et alimentaire.**

(30) Priorité: **28.01.82 FR 8201325**

(43) Date de publication de la demande:
**10.08.83 Bulletin 83/32**

(45) Mention de la délivrance du brevet:
**11.03.87 Bulletin 87/11**

(84) Etats contractants désignés:
**AT BE CH DE FR IT LI LU NL**

(56) Documents cités:
**FR - A - 1 570 435**
**FR - A - 2 068 515**
**FR - A - 2 161 867**
**FR - A - 2 290 497**

**CHEMICAL ABSTRACTS, vol. 91, no. 11, 10 septembre 1979, page 380, no. 86874b, Columbus, Ohio, USA, M.M. BASHOR: "Cell culture. Dispersion and disruption of tissues"**

(73) Titulaire: **LABORATOIRE VAILLANT DEFRESNE,**
**65-77 rue Falguière, F-75739 Paris Cedex 15 (FR)**

(72) Inventeur: **Jean, Daniel, Rue de la Chaussade, 63270 Vic le Comte (FR)**

(74) Mandataire: **Ores, Irène et al, CABINET ORES 6, Avenue de Messine, F-75008 Paris (FR)**

ACTORUM AG

### Description

La présente invention est relative à des perfectionnements apportés aux procédés de préparation de suspensions de cellules fraîches stabilisées destinées à l'usage pharmaceutique, cosmétique ou alimentaire.

Les cellules, qu'elles soient d'origine végétale, animale, humaine ou microbienne, constituent de véritables réservoirs des divers organites et principes actifs et sont toujours très utilisées soit telles quelles, soit sous forme d'extraits dans de nombreuses préparations et notamment à usage pharmaceutique.

Les procédés pour obtenir ces préparations cellulaires comportent deux grands écueils:

Le premier écueil est constitué par le broyage lui-même: s'il est pratiqué sans précautions, il a pour effet immédiat de mettre en contact des sites qui, à l'état normal, étaient géographiquement séparés et, de ce fait, favoriser l'action d'enzymes sur des substrats.

Un autre écueil est constitué par divers processus physiques et chimiques mis en œuvre (tels que température, pH, solvants, etc.) qui tous ont une influence plus ou moins néfaste sur les principes actifs et l'équipement enzymatique de la cellule.

Enfin, il est très important de stabiliser les différentes préparations cellulaires afin d'éviter la dégradation des différents principes actifs avec le temps.

Les différents procédés tels que pratiqués actuellement, et ceci malgré les grandes précautions prises, n'ont pu éviter les différents écueils et fournissent des préparations cellulaires transformées et notablement appauvries en différents principes actifs.

Ainsi par exemple, même la lyophilisation qui permet un séchage rapide à basse température outre qu'elle constitue une technique coûteuse, provoque la perte par évaporation des divers principes volatils tels que les huiles essentielles, certaines amines et divers arômes. Elle provoque même certaines altérations dues à la phase ultime du séchage fait à une température voisine de 20 °C.

Ainsi également par exemple, il y a des risques sérieux de dégradation par l'extraction aux divers solvants, très largement utilisée et qui fait appel à la solubilisation des substances contenues dans la cellule. En outre, cette solubilisation n'est pas complète, même si l'on fait appel à toute une série de solvants successifs. L'évaporation du solvant (ou même l'extraction elle-même), se fait parfois à chaud et est donc dangereuse pour les divers principes actifs. De plus, les résidus des divers solvants s'avèrent souvent toxiques.

La présente invention s'est par conséquent fixé pour but de pourvoir à un procédé de préparation de suspensions de cellules fraîches, qui répond mieux aux nécessités de la pratique que les procédés visant au même but et antérieurement connus, notamment en ce qu'il préserve la quasi-totalité des principes actifs de la cellule tout en étant économique et facile à mettre en œuvre.

La présente invention a pour objet un procédé de préparation de suspensions de cellules fraîches stabilisées, caractérisé en ce qu'il comporte les étapes suivantes:

a) nettoyage poussé de la matière première (plante ou organe de l'animal), opéré 24 heures au plus tard après la récolte;

b) broyage à une température inférieure à 0 °C jusqu'à l'obtention d'une granulométrie inférieure à environ 100 µm;

c) un premier broyage colloïdal ou micronisation en présence d'un solvant miscible à l'eau à une température inférieure à –5 °C et de préférence inférieure à –15 °C afin d'obtenir des particules de granulométrie inférieures à 50 µm;

d) un deuxième broyage colloïdal ou micronisation opéré dans les mêmes conditions que le premier broyage colloïdal, mais à un débit deux fois plus faible que le premier broyage colloïdal, afin d'obtenir des particules de granulométrie moyenne de 10 µm, mais toujours inférieure à 20 µm;

e) conditionnement de la suspension de cellules fraîches à une température inférieure à 30 °C dans un flaconnage opaque, à l'abri de la chaleur.

Conformément à l'invention, le solvant utilisé est pris dans le groupe qui comprend l'éthanol, le glycérol, le monopropylène-glycol, le dipropylène-glycol, le tripropylène-glycol, l'éthyl-diglycol, l'éthylène-glycol et l'isopropylidène-glycérol.

Selon un mode de réalisation avantageux du procédé objet de la présente invention, le broyage opéré en étape b) du procédé est également effectué en présence d'un solvant choisi parmi ceux mentionnés ci-dessus et en quantité M telle que M soit compris entre RP et 5 RP,

M étant le poids de solvant utilisé (pur ou dilué) en litres;

R étant la teneur en matière sèche de la matière première; et

P étant le poids de la matière première en kg.

Conformément à l'invention, la quantité M' du solvant (pur ou dilué) utilisé lors des broyages colloïdaux ou micronisations est comprise entre 10 RP et 100 RP, R et P ayant la même signification que ci-dessus. Il est bien entendu que le solvant utilisé sera le même que celui éventuellement mis en œuvre à l'étape b) du procédé et l'on se contentera d'ajuster la quantité M pour obtenir M' qui sera compris entre 10 RP et 100 RP.

Conformément à l'invention, la quantité d'eau de dilution éventuellement ajoutée au solvant sera calculée de manière à ce que le point de congélation du solvant dilué soit toujours inférieur à 15°C.

Selon un mode de réalisation particulièrement avantageux du procédé objet de la présente invention, on procède à un troisième, quatrième,... nième broyage colloïdal ou micronisation, s'il s'avérait que la granulométrie inférieure à 20 µ n'était pas atteinte.

Suivant une modalité particulière de ce mode de réalisation, le débit de chacun des broyages colloïdaux successifs sera réduit entre 1,5 à 2 fois.

Les exemples de mise en œuvre qui sont décrits ci-après illustrent l'invention.

Exemples de préparation:

EXEMPLE I

Préparation d'une suspension éthanolique de cellules de feuilles de Cynara scolymus destinée à l'usage pharmaceutique.

Les feuilles d'artichaut ou Cynara scolymus sont employées en thérapeutique pour traiter des désordres hépatiques. Les feuilles aussitôt après la cueillette sont passées sous un courant d'eau froide, puis soigneusement égouttées et placées au congélateur à −85 °C.

Cinq échantillons de 10 g environ sont broyés dans un mixer de laboratoire, placés à l'étuve à 120 °C et pesés à poids constant pour déterminer R, soit le taux de matières sèches. On prélève 10 kg de feuilles fraîches congelées et on les place dans un broyeur à couteaux d'une contenance de 60 litres.

On pèse 10 kg d'éthanol pur que l'on dilue par 18 kg d'eau distillée, et on le verse dans une cuve agitée lentement puis on abaisse la température à −50 °C par une addition d'azote liquide. Quand la température est atteinte, on verse l'éthanol dilué refroidi dans le broyeur à couteaux et l'on broie 2 minutes à 3000 tpm.

La température de la suspension est alors abaissée si besoin est à −25 °C puis on opère un premier broyage colloïdal à un débit de 1,5 litre par seconde. La température est à nouveau portée à −25 °C et on réalise un deuxième broyage à un débit de 0,75 litre par seconde.

Après mesure de la granulométrie au micromètre sous microscope, on décide de l'opportunité d'un ou de plusieurs broyages supplémentaires dans les mêmes conditions. Si la granulométrie est atteinte, c'est-à-dire si aucune particule n'est supérieure à 20 µm et si la moyenne se situe aux alentours de 10 µm, alors on conditionne dans un récipient opaque et on stocke à +10 °C.

On obtient ainsi 40 kg de suspension à 30% d'éthanol pur en poids et 5% de matière sèche totale.

Analyse du produit obtenu:
a) Chromatographie sur couches minces des flavones:

Support: Silicagel G
Solvant: Acétate d'éthyle, acide formique, eau: 8/1/1
Révélateur: Chlorure d'aluminium à 5% dans le méthanol pur
Observation des spots sous lumière U. V. à 366 nm
Résultats: Cynaroside Rf = 0,81
Scolymoside Rf = 0,53
Lutéoline Rf = 0,88

b) Dosage des flavones par la méthode de WILSON (J. Am. Chem. Soc. 1939, 61, p. 2303):

100 g de préparation sont soumis à une extraction totale dans un extracteur Soxlhet par de l'éthanol aqueux à 30 °C. On peut ainsi évaluer la quantité réelle de principes flavoniques contenus dans la préparation, en effectuant le dosage sur la solution extractive. La teneur en flavones est égale à celle de la plante fraîche ±5%.

c) Dosage des ortho diphénols:

Sur la même solution extractive que pour le dosage des flavones, on réalise le dosage suivant:

Réactif:
Nitrite de sodium 10 g
Molybdate de sodium 10 g
Eau distillée 100 ml
Témoin:
Solution à doser 0,5 ml
HCl 0,5 N 0,5 ml
Eau distillée 14 ml
Essai:
Solution à doser 0,5 ml
HCl 0,5 N 0,5 ml
Eau distillée 13,5 ml
Réactif 0,5 ml

On attend dix minutes à la température du laboratoire et on ajoute 2 ml de soude à 40% dans tous les tubes. On lit la densité optique (D. O.) à 515 nm.

La valeur lue pour la préparation est égale à celle de la plante fraîche ±5%.

d) Dosage des oxydases:

50 g de préparation homogénéisée sont filtrés sur un filtre en verre fritté n° 3. La partie insoluble, lavée une fois par 100 ml d'eau distillée froide (environ 5 °C) est mise en suspension dans une solution à 0,1% de paraphénylènediamine tamponnée à pH 5,6, et placée au bain-marie à 40 °C pendant 30 minutes. La D. O. est lue après filtration sur papier à 483 nm contre un témoin réalisé dans les mêmes conditions mais sans insoluble. La valeur trouvée est au moins égale à 70% de la valeur trouvée pour un poids de feuilles fraîches correspondant à une quantité de matière sèche égale à celle de la préparation soumise au dosage.

e) Contrôles physiques:
− Mesure de la granulométrie: aucune particule ne devra posséder une taille supérieure à 20 µm et la moyenne devra se situer aux alentours de 10 µm;
− Mesure de la matière sèche totale: on pèse exactement environ 1 g de suspension parfaitement homogénéisée que l'on place à l'étuve à 120 °C. On le pèse à poids constant. Le pourcentage de matière sèche sera égal à la valeur théorique ±2%.

EXEMPLE II

Préparation d'une suspension hydroglycolique de cellules de bulbes frais de Lilium candidum destinée à l'usage cosmétologique.

Immédiatement après la récolte, les bulbes de lis sont triés et écaillés soigneusement, puis lavés rapidement sous un courant d'eau froide, égouttés sur un grillage en acier inoxydable puis placés au congélateur à −85 °C.

On détermine le taux R de matière sèche sur 5 échantillons comme dans l'exemple I.

On prélève une quantité P de bulbes frais congelés correspondant à un poids de 4 kg de matière sèche et on la place dans un broyeur à couteaux d'une contenance de 60 litres. On broie d'abord à une vitesse de 1500 tpm pour casser les plus grosses masses congelées puis on opère un broyage à 3000 tpm pendant environ 2 minutes. On doit alors obtenir une poudre fine. Si la poudre a tendance à s'agglomérer en masses molles, on injecte de l'azote liquide afin de se placer à une température de −25°C et on opère un autre broyage à 3000 tpm pendant 30 secondes.

Dans une cuve agitée contenant 20 kg de monopropylène-glycol et un volume V d'eau distillée tel que V = 20 − P, porté à une température de −25°C par addition d'azote liquide on place la plante congelée et broyée. Après stabilisation de la température, on ajoute à nouveau de l'azote liquide pour atteindre −25°C et on réalise un premier broyage colloïdal à un débit de 2 litres par seconde. Puis on effectue, comme dans l'exemple I, d'autres broyages colloïdaux afin d'atteindre une granulométrie moyenne de 10 μm et maximale de 20 μm.

On conditionne dans des récipients opaques et l'on stocke à +10 °C.

Contrôle du produit terminé:

On procède tout d'abord à l'identification microscopique, à la mesure de la granulométrie, au dosage de l'eau par entraînement azéotropique, à la mesure de la matière sèche totale, à la recherche des métaux lourds par la méthode de la Pharmacopée; puis on effectue les dosages suivants:

1) Dosages des mucilages:

100 g de suspension sont portés dans un sac en acétate de cellulose que l'on ferme de façon étanche. On place le sac dans un bac dans lequel circule de l'eau à 20 °C. Puis le sac est sorti de l'eau, ouvert, et vidé de son contenu dans un extracteur Soxlhet où l'on effectue une extraction par l'eau jusqu'à ce que la solution extractive ne laisse plus aucun résidu à l'évaporation.

Sur la solution extractive diluée au 1/100 on réalise le dosage suivant:

Réactif:

| | |
|---|---|
| Anthrone: | 200 mg |
| H$_2$SO$_4$ concentré: | 200 ml |
| Eau distillée: | 100 ml |

Témoin:

| | |
|---|---|
| Réactif: | 10 ml |
| Eau distillée: | 1 ml |

Essai:

| | |
|---|---|
| Réactif: | 10 ml |
| Solution à doser: | 1 ml |

On place tous les tubes pendant 15 minutes au bain marie bouillant et on lit la D. O. à 620 nm.

L'étalonnage est réalisé par une gamme de glucose qui sert de référence pour l'expression des résultats.

La valeur trouvée est égale à celle des bulbes frais ±10%.

2) Dosage des oxydases:

On opère comme dans l'exemple I. La valeur trouvée est au moins égale à 60% de la valeur trouvée pour les bulbes frais.

EXEMPLE III:

Préparation d'une suspension hydroglycérolique de cellules de noix (cônes) de Cupressus sempervirens fraîches à usage pharmaceutique.

La noix de Cyprès possède une nette activité veinotrope utilisable en thérapeutique dans les troubles circulatoires périphériques.

Les noix ne peuvent être broyées en présence d'un trop grand volume de solvant pour des raisons mécaniques. Elles sont cueillies, rincées à l'eau froide et essuyées dans un linge.

Le taux de matière sèche est déterminé par entraînement azéotropique au xylène car la présence d'huile essentielle rend la méthode par dessication invalide par défaut.

Un poids P de noix fraîches correspondant à un poids p = 2 kg de matière sèche est immergé dans une cuve sous agitation lente mais constante et contenant 6 kg de glycérol pur et un volume V$_1$ d'eau distillée tel que:

$$V_1 = \frac{20-P}{3}.$$

Le solvant est porté à une température de −55°C par addition d'azote liquide. Quand la température est stabilisée, on transfère les noix et le solvant dans un broyeur à couteaux de 60 litres de capacité. On effectue tout d'abord un broyage à 1500 tpm pendant 30 secondes puis un broyage à 3000 tpm pendant 4 minutes.

Dans la cuve agitée, on place alors 14 kg de glycérol pur et un volume V$_2$ d'eau distillée tel que:

$$V_2 = \frac{2\,(20-P)}{3}$$

Ce mélange est porté à −55 °C par de l'azote liquide et transféré dans le broyeur à couteaux.

On effectue alors un troisième broyage à 3000 tpm pendant 2 minutes, puis la température est éventuellement ajustée à −25 °C par de l'azote liquide et on réalise un premier broyage colloïdal à un débit de 1 litre par minute.

On ajuste de nouveau la température à −25 °C et on effectue un deuxième broyage colloïdal à un débit de 0,5 litre par minute.

Si la granulométrie de 10 μm en moyenne et de 20 μm au maximum n'est pas atteinte, on opère comme dans les exemples I et II.

Le produit est ensuite conditionné dans des récipients étanches aux gaz et opaques à la lumière. On stocke à 15 °C.

Contrôle du produit terminé:

On procède tout d'abord à l'identification microscopique, à la mesure de la granulométrie et à la mesure de la matière sèche totale.

Puis on effectue les dosages suivants:

1) Dosage des huiles essentielles (par la méthode de la Pharmacopée), on doit trouver une valeur égale à celle de la plante fraîche ±5%.

2) Chromatographie sur couche mince des leucoanthocyanes:

10 g de suspension sont hydrolysés pendant une heure par de l'acide chlorhydrique normal au bain marie bouillant à reflux. Puis on extrait une fois par du n-butanol après avoir ajouté 50 ml d'eau distillée. La phase butanolique est ensuite chromatographiée:

Support:       Cellulose MN 300
Solvant:       Acide acétique, acide chlorhydrique
               concentré, eau distillée: 30-3-10
Observation directe à la lumière ordinaire
Résultats:     Un spot rose à Rf 0,40
               Un spot brun-rose à Rf 0,98

3) Dosage des tanins catéchiques:

100 g de suspension sont extraits par de l'éthanol à 50 °C dans un extracteur Soxlhet.

La solution extractive est concentrée sous pression réduite à 50 °C jusqu'à évaporation totale de l'eau et de l'alcool. Puis on traite le résidu glycérolique par 200 ml de formol à 30% et 100 ml d'acide chlorhydrique concentré. Le mélange est placé au bain marie bouillant à reflux sous un léger courant d'azote. Après polymérisation, on filtre sur verre fritté n° 3 taré. On lave abondamment par de l'eau distillée, puis on sèche à l'étuve à 120 °C à poids constant.

On trouve une valeur égale à celle trouvée pour la plante fraîche ±10%.

4) Dosage des oxydases:

On opère comme dans les exemples I et II. Le résultat doit être au moins égal à 70% de la valeur trouvée pour la plante fraîche.

Il résulte de la description qui précède que quels que soient les modes de mise en œuvre, de réalisation et d'application adoptés on obtient un procédé de préparation de suspensions de cellules fraîches (végétales, animales ou microbiennes) qui présentent par rapport aux procédés visant au même but antérieurement connus des avantages importants et notamment l'avantage d'obtenir des cellules ayant conservé pratiquement intacts tous les principes actifs de la cellule vivante.

**Revendications**

1. Procédé de préparation de suspensions de cellules fraîches stabilisées caractérisé en ce qu'il comporte les étapes suivantes:

a) nettoyage poussé de la matière première (plante ou organe de l'animal), opéré 24 heures au plus tard après la récolte;

b) broyage à une température inférieure à 0 °C jusqu'à l'obtention d'une granulométrie inférieure à environ 100 µm;

c) un premier broyage colloïdal ou micronisation en présence d'un solvant miscible à l'eau à une température inférieure à −5 °C et de préférence inférieure à −15 °C, afin d'obtenir des particules de granulométrie inférieure à 50 µm, ledit solvant est pris dans le groupe qui comprend l'éthanol, le glycérol, le monopropylène-glycol, le dipropylène-glycol, le tripropylène-glycol, l'éthyl-diglycol, l'éthylène-glycol et l'isopropylidène-glycérol, et la quantité M' du solvant (pur ou dilué) utilisé lors des broyages colloïdaux ou micronisations sera comprise entre 10 RP et 100 RP, R étant la teneur en matière sèche de la matière première et P étant le poids de la matière première en kg;

d) un deuxième broyage colloïdal ou micronisation opéré dans les mêmes conditions que le premier broyage colloïdal, mais à un débit deux fois plus faible que le premier broyage colloïdal, afin d'obtenir des particules de granulométrie moyenne de 10 µm, mais toujours inférieure à 20 µm;

e) conditionnement de la suspension de cellules fraîches à une température inférieure à 30 °C dans un flaconnage opaque, à l'abri de la chaleur.

2. Procédé selon la revendication 1, caractérisé en ce que le broyage opéré en étape b) du procédé est également effectué en présence d'un solvant choisi dans le groupe qui comprend l'éthanol, le glycérol, le monopropylène-glycol, le dipropylène-glycol, le tripropylène-glycol, l'éthyl-diglycol, l'éthylène-glycol et l'isopropylidène-glycérol et en quantité M telle que M soit compris entre RP et 5 RP, M étant le poids de solvant utilisé (pur ou dilué) en litres, R et P ayant la même signification que ci-dessus.

3. Procédé selon l'une quelconque des revendications 1 et 2, caractérisé en ce que la quantité d'eau de dilution éventuellement ajoutée au solvant sera calculée de manière à ce que le point de congélation du solvant dilué soit toujours inférieur à −15 °C.

4. Procédé selon la revendication 1, caractérisé en ce que le débit de chacun des broyages colloïdaux successifs sera réduit entre 1,5 à 2 fois.

5. Suspension de cellules (végétales, animales ou microbiennes) obtenue suivant l'une quelconque des revendications 1 à 4.

6. Application de cellules selon la revendication 5 à usage pharmaceutique, cosmétologique ou alimentaire.

**Claims**

1. Process for the preparation of stabilized fresh cell suspensions characterized in that it comprises the following steps:

a) thorough cleaning of the raw material (plant or animal organ), carried out 24 hours at the latest after gathering;

b) grinding at a temperature below 0 °C until a granulometry below about 100 µm has been obtained;

c) a first colloidal grinding or micronisation in the presence of a water-miscible solvent at a temperature below −5 °C and preferably below −15 °C, in order to obtain particles of granulometry below 50 µm, said solvent is taken from the group which comprises ethanol, glycerol monopropylene-glycol, dipropylene-glycol, tripropylene-glycol, ethyl-diglycol, ethylene-glycol and isopropylidene-glycerol, and the amount M′ of the solvent (pure or dilute) used in the colloidal grindings or micronisations is comprised between 10 RP and 100 RP, R being the content of dry matter of the raw material and P being the weight of the raw material in kg;

d) a second colloidal grinding or micronisation performed under the same conditions as the first colloidal grinding, but at a flow rate twice as low as the first colloidal grinding, in order to obtain particles of average granulometry of 10 µm, but always less than 20 µm;

e) packaging of the fresh cell suspension at a temperature below 30°C in opaque bottles, protected from heat.

2. Process according to claim 1, characterized in that the grinding performed in step b) of the process is also carried out in the presence of a solvent selected from the group which comprises ethanol, glycerol, monopropylene-glycol, dipropylene-glycol, tripropylene-glycol, ethyl-diglycol, ethylene-glycol and isopropylidene-glycerol and in an amount M such that M is comprised between RP and 5 RP, M being the weight of solvent used (pure or dilute) in liters, R and P having the same meaning as above.

3. Process according to any of claims 1 and 2, characterized in that the amount of dilution water possibly added to the solvent is calculated so that the freezing point of the diluted solvent is always less than −15 °C.

4. Process according to claim 1, characterized in that the flow rate of each of the successive colloidal grindings is reduced between 1.5 to 2 times.

5. Cell suspension (vegetable, animal or microbial) obtained according to any one of claims 1 to 4.

6. Application of cells according to claim 5 to pharmaceutical, cosmetological or food use.

**Patentansprüche**

1. Verfahren zur Herstellung von frischen, stabilisierten Zellsuspensionen, dadurch gekennzeichnet, dass es folgende Stufen umfasst:

a) Kräftige Reinigung des Ausgangsmaterials (Pflanze oder tierisches Organ), durchgeführt spätestens 24 Stunden nach der Ernte;

b) Zerkleinerung bei einer Temperatur unter 0 °C bis zur Erzielung einer Granulometrie unter etwa 100 µm;

c) eine erste kolloidale Zerkleinerung oder Mikronisierung in Anwesenheit eines mit Wasser mischbaren Lösungsmittels bei einer Temperatur unter −5 °C und vorzugsweise unter −15 °C, um Teilchen mit einer Granulometrie unter 50 µm zu erhalten, wobei das Lösungsmittel ausgewählt wird aus der Gruppe von Ethanol, Glyzerin, Monopropylenglykol, Dipropylenglykol, Tripropylenglykol, Ethyldiglykol, Ethylenglykol und Isopropylidenglyzerin, und die Menge M′ des Lösungsmittels (rein oder verdünnt), die während der kolloidalen Zerkleinerungen oder Mikronisierungen verwendet wird, zwischen 10 RP und 100 RP liegt, wobei R der Gehalt des Trockenmaterials des Ausgangsmaterials ist und P das Gewicht des Ausgangsmaterials in kg ist;

d) eine zweite kolloidale Zerkleinerung oder Mikronisation, durchgeführt unter den gleichen Bedingungen wie die erste kolloidale Zerkleinerung, jedoch mit einem zweifach kleineren Durchsatz als die erste kolloidale Zerkleinerung, um Teilchen mit einer mittleren Granulometrie von 10 µm, jedoch immer unter 20 µm, zu erhalten;

e) Konditionieren der Suspension frischer Zellen bei einer Temperatur unter 30 °C in undurchsichtigen Flaschen unter Ausschluss von Wärme.

2. Verfahren nach Anspruch 1, dadurch gekennzeichnet, dass die in der Stufe b) des Verfahrens durchgeführte Zerkleinerung auch in Anwesenheit eines Lösungsmittels durchgeführt wird, das ausgewählt wird aus der Gruppe von Ethanol, Glyzerin, Monopropylenglykol, Dipropylenglykol, Tripropylenglykol, Ethyldiglykol, Ethylenglykol, Isopropyliden-glyzerin und in einer derartigen Menge M, dass M zwischen RP und 5 RP liegt, wobei M das Gewicht des verwendeten Lösungsmittels (rein oder verdünnt) in Liter ist und R und P die gleiche Bedeutung wie vorstehend haben.

3. Verfahren nach einem der Ansprüche 1 und 2, dadurch gekennzeichnet, dass die Menge an Wasser zur Verdünnung, die gegebenenfalls zu dem Lösungsmittel zugefügt wird, so berechnet wird, dass der Gefrierpunkt des verdünnten Lösungsmittels immer unter −15 °C liegt.

4. Verfahren nach Anspruch 1, dadurch gekennzeichnet, dass der Durchsatz jeder der aufeinanderfolgenden kolloidalen Zerkleinerungen zwischen 1,5- und 2-fach verringert wird.

5. Zellsuspension (pflanzlich, tierisch oder mikrobiell), erhalten nach einem der Ansprüche 1 bis 4.

6. Verwendung der Zellen nach Anspruch 5 für pharmazeutische, kosmetische und Nahrungsmittel-Zwecke.